# FASCICULE DE BREVET EUROPEEN

(11) **EP 1 585 526 B1**
(45) Date de publication et mention de la délivrance du brevet: **14.07.2010**
(21) Numéro de dépôt: 04704259.3
(22) Date de dépôt: 22.01.2004
(51) Int. Cl.: A61K 31/575, A61P 31/10, A61P 25/28, A61P 35/00

(54) **NOUVELLE UTILISATION DE LA MIFEPRISTONE ET DE SES DERIVES COMME MODULATEURS DE LA VOIE DE SIGNALISATION DES PROTEINES HEDGEHOG ET SES APPLICATIONS**
NEUE VERWENDUNG DES MIFEPRISTON UND DESSEN DERIVATEN ZUR MODULIERUNG DES "HEDGEHOG"-PROTEINSIGNALWEGES UND DESSEN ANWENDUNGEN
NOVEL USE OF MIFEPRISTONE AND DERIVATIVES THEREOF AS HEDGEHOG PROTEIN SIGNALLING PATHWAY MODULATORS AND APPLICATIONS OF SAME

(30) Priorité: 22.01.2003 FR 0300646
(43) Date de publication de la demande: 19.10.2005
(73) Titulaire: CENTRE NATIONAL DE LA RECHERCHE SCIENTIFIQUE (CNRS), 75794 Paris Cedex 16 (FR)
(72) Inventeur: RUAT, Martial, F-91400 ORSAY (FR); TRAIFFORT, Elisabeth, F-75013 Paris (FR); FAURE, Hélène, F-91190 Gif-sur-Yvette (FR)
(74) Mandataire: Orès, Bernard
(86) Numéro de dépôt international: PCT/FR2004/000151
(87) Numéro de publication internationale: WO 2004/067550

(56) Documents cités:
- WO-A-00/24390
- WO-A-98/27986
- WO-A-98/48784
- WO-A-99/59596
- US-A- 4 386 085
- KOIDE S S: "Mifepristone. Auxiliary therapeutic use in cancer and related disorders." THE JOURNAL OF REPRODUCTIVE MEDICINE. JUL 1998, vol. 43, no. 7, juillet 1998 (1998-07), pages 551-560, XP008031509 ISSN: 0024-7758
- WEISS B D: "RU 486. The progesterone antagonist." ARCHIVES OF FAMILY MEDICINE. JAN 1993, vol. 2, no. 1, janvier 1993 (1993-01), pages 63-69 ; disc, XP008031514 ISSN: 1063-3987
- HAAK H R ET AL: "Successful mifepristone treatment of recurrent, inoperable meningioma." LANCET. 14 JUL 1990, vol. 336, no. 8707, 14 juillet 1990 (1990-07-14), pages 124-125, XP001189603 ISSN: 0140-6736
- BONELLI R M: "[Mifepristone (RU 486)]" DISKUSSIONSFORUM MEDIZINISCHE ETHIK. 1992, no. 6-7, 1992, pages XXXVIII-XLIII, XP008031506
- OLSON J J ET AL: "Hormonal manipulation of meningiomas in vitro." JOURNAL OF NEUROSURGERY. JUL 1986, vol. 65, no. 1, juillet 1986 (1986-07), pages 99-107, XP008031507 ISSN: 0022-3085
- FRYER C J ET AL: "Selective activation of the glucocorticoid receptor by steroid antagonists in human breast cancer and osteosarcoma cells." THE JOURNAL OF BIOLOGICAL CHEMISTRY. 9 JUN 2000, vol. 275, no. 23, 9 juin 2000 (2000-06-09), pages 17771-17777, XP002285331 ISSN: 0021-9258
- BEHL ET LA: "Protection Against Oxidative Stress-induced Neuronal Cell Death- A novel Role for RU486" EUROPEAN JOURNAL OF NEUROSCIENCE, OXFORD UNIVERSITY PRESS, GB, vol. 9, no. 5, 1 mai 1997 (1997-05-01), pages 912-920, XP002080517 ISSN: 0953-816X
- BELANOFF J K ET AL: "SLOWING THE PROGRESSION OF COGNITIVE DECLINE IN ALZHEIMER'S DISEASE USING MIFEPRISTONE" JOURNAL OF MOLECULAR NEUROSCIENCE, BIRKHAEUSER, CAMBRIDGE, MA, US, vol. 19, no. 1/2, août 2002 (2002-08), pages 201-206, XP009030048 ISSN: 0895-8696
- GETTYS ET AL: "Ru-486 (mifepristone) ameliorates diabetes but does not correct deficient beta-adrenergic signalling in adipocytes from mature C57Bl/6J-ob/ob mice" INTERNATIONAL JOURNAL OF OBESITY, NEWMAN PUBLISHING, LONDON, GB, vol. 21, no. 10, octobre 1997 (1997-10), pages 865-873, XP002117766 ISSN: 0307-0565

## Description

La présente invention est relative à une nouvelle utilisation de la mifépristone et de ses dérivés comme modulateurs de la voie de signalisation des protéines Hedgehog, ainsi qu'à ses applications pour la préparation d'un médicament destiné au traitement de pathologies impliquant un dysfonctionnement tissulaire lié à une dérégulation de cette voie.

La voie de signalisation des protéines Hedgehog joue un rôle essentiel dans le développement de nombreux tissus embryonnaires, en particulier au niveau du système nerveux et serait impliquée dans le maintien et la réparation tissulaire chez l'adulte.

Le gène Hedgehog (*Hh*)*,* isolé chez la drosophile puis chez les vertébrés code pour une famille de protéines de signalisation sécrétées et très conservées, les protéines Hedgehog (Hh), qui jouent le rôle de morphogènes dans de nombreux tissus (Pour une revue voir : Ingham et al., Genes Dev., 2001, 15, 3059-3087 ; Marti et al., Trends Neurosci, 2002, 25, 89-96 ; Weschler et al., Annu Rev Neurosci, 2001, 24, 385-428). Les protéines Hh sont synthétisées sous la forme de précurseurs immatures d'environ 45 kDa autoprocessés en une forme amino-terminale active (HhNp pour *N-terminal processed domain*), doublement modifiée par l'addition de deux lipides : un résidu cholestérol (liaison ester carboxy-terminale) et un palmitate (liaison amide amino-terminale). Chez les mammifères, il existe trois familles de protéines Hh : Sonic (Shh), Indian (Ihh) et Desert (Dhh). Shh participe à la ventralisation du tube neural en spécifiant le phénotype précoce de plusieurs types neuronaux le long de la ligne médiane ventrale (motoneurones de la moelle épinière, neurones dopaminergiques ou cholinergiques) et en induisant la génération des précurseurs oligodendrocytaires à partir de la moelle épinière ventrale. Par ailleurs, Shh induit la survie des neurones gabaergiques et dopaminergiques, oriente le devenir des précurseurs sérotoninergiques et prévient la mort des neurones dopaminergiques provoquée par la toxine MPP. Il induit enfin la prolifération des précurseurs des cellules granulaires dans le cervelet post-natal précoce. Les autres membres de la famille Hedgehog, participent quant à eux, respectivement au développement du tissu osseux (Ihh), des testicules et des nerfs périphériques (Dhh).

Plus récemment la voie Shh a été identifiée dans le cerveau adulte, où la forme active amino-terminale de la molécule est exprimée dans de nombreuses régions du système nerveux mature, à un niveau plus élevé que celui rencontré au cours de la période post-natale précoce (Traiffort et al., Eur. J. Neurosci. : 1999 : 11, 3199-3214 et 2001, 14, 839-850). Bien que les rôles de Shh chez l'adulte ne soient pas complètement élucidés, il est d'abord apparu, à l'image d'autres molécules neurotrophiques, comme un facteur capable de promouvoir la survie et le maintien du phénotype des cellules du système nerveux (Reilly et al., Mol. Cell. Neurosci., 2002, 19, 88-96 ; Charytoniuk et al., Eur. J. Neurosci., 2002, 16, 2351-2357). Dans des conditions pathologiques, telles qu'un modèle de la maladie de Parkinson ou un modèle de neuropathie périphérique, Shh est capable, de préserver les projections axonales des neurones dopaminergiques dans le striatum ou d'améliorer le temps nécessaire à la récupération motrice consécutive à l'écrasement du nerf sciatique (Tsuboi et al., Exp. Neurol., 2002, 173, 95-104 ; Pepinski et al., J. Pharm. Sci., 2002, 91, 371-387)..

La réponse cellulaire au morphogène Hedgehog est contrôlée par les produits d'expression du gène *Patched* (*Ptc*), un gène suppresseur de tumeurs, et du proto-oncogène *Smoothened* (*Smo*) ; toutefois, le mécanisme exact de la régulation de la voie Hedgehog n'est pas complètement élucidé. Chez les mammifères il existe deux gènes Patched codant respectivement pour Ptc1 et Ptc2, des glyco-protéines à 12 domaines transmembranaires, homologues à des transporteurs bactériens. Le produit du gène *Smo* qui code pour une protéine de la famille des récepteurs couplés aux protéines G, ne possède aucun ligand endogène connu. En l'absence de Hedgehog, Ptc bloquerait l'activité constitutive de Smo. La liaison de Hedgehog à Ptc lèverait cette inhibition et permettrait la transduction du signal par l'intermédiaire de Smo. Le mécanisme de régulation de l'activité de Smo par Ptc, chez les mammifères, pourrait faire intervenir une molécule transportée par Ptc et interagissant avec Smo (Taipale et al., Nature, 2002, 418, 892-896). L'activation des facteurs de transcription Gli est impliquée dans la cascade d'événements résultant de l'activité de Smo. La protéine transmembranaire de type I, HIP (Hedgehog Intercating Protein), constitue un autre récepteur des molécules Hedgehog qu'il lie avec une affinité comparable à celle de Ptc ; HIP a été proposé comme un régulateur négatif de la voie (Ingham et al., précité ; Ho et al., Curr. Opin. Neurobiol., 2002, 12, 57-63; Taipale et al., Nature, 2001, 411, 349-354). En outre, les produits du gène *dispatched* (*disp*), notamment DispA seraient impliqués dans le relargage et l'accumulation dans le milieu extracellulaire des protéines Hedgehog sous forme soluble (Ma et al., Cell, 2002, 111, 63-75).

Des dysfonctionnements de la voie de signalisation Shh ont été associés à de nombreux cancers, en particulier à la suite de la caractérisation de *Ptc* comme gène suppresseur de tumeur. En effet, des mutations inactivatrices de *Ptc* sont associées au Syndrome de Gorlin ou naevomatose basocellulaire, une maladie autosomale dominante caractérisée par des malformations cranofaciales et cérébrales, mais surtout par une incidence élevée de diverses tumeurs, plus particulièrement des carcinomes basocellulaires au niveau cutané et des médulloblastomes, au niveau cérébral. Des souris hétérozygotes pour le gène *Ptc* développent des tumeurs du cervelet suggérant qu'une modification de la voie Shh est à l'origine de ces tumeurs (Goodrich et al., Science, 1997, 277, 1109-1113).

Des mutations des gènes *Ptc* ou *Smo* humains sont également observées dans des tumeurs primitives neuroectodermales du système nerveux central, principalement des médulloblastomes (30 % des cas), mais aussi dans des formes sporadiques de carcinomes basocellulaires (40 % et 20 %, respectivement pour *Ptc* et *Smo*)*.* En outre, des mutations de Shh (H133Y) sont également associées, à des carcinomes basocellulaires. Les mutations de Smo qui concernent principalement deux acides aminés situés dans le septième domaine hydrophobe du récepteur (W535L et S533N), induisent une activation constitutive de la voie qui échappe au contrôle négatif de Ptc. En revanche, celles de Ptc conduisent à une diminution de l'inhibition qu'il exerce sur Smo en l'absence de Shh. Dans les deux cas, une activation de la voie Shh en résulte et conduit à une puissante activité mitogénique démontrée dans des cultures de précurseurs de cellules granulaires de cervelet en développement, et à un blocage de l'étape terminal de différenciation de ces neuroblastes (Traiffort et al., Eur. J ; Neurosci., 1999, précité ; Charytoniuk et al., J. Physiol. Paris, 2002, 96, 9-16 ; Dahmane et al., development, 1999, 126, 3089-3100 ; Wallace et al., Curr. Biol., 1999, 22, 103-114 ; Weshler-Reya et al., Neuron., 1999, 22, 103-114). De même l'expression de Smo portant une de ces mutations dans des souris transgéniques, conduit à la présence de carcinomes basocellulaires, ce qui démontre l'implication directe de Smo dans le développement de ces tumeurs (Xie et al., Nature, 1998, 391, 90-92).

En dehors des carcinomes basocellulaires et des médulloblastomes, d'autres types de tumeurs ont été associées à un défaut de la voie de signalisation Hedgehog ; la localisation de ces tumeurs est étroitement corrélée aux sites d'expression des composantes de la voie au cours du développement embryonnaire. A titre d'exemple non-limitatif on peut citer : des cancers du sein et des méningiomes associés à des mutations de Ptc, et des glioblastomes associés à des mutations de Gli.

Du fait du rôle crucial de la voie de signalisation des protéines Hedgehog dans de nombreux processus physiologiques et par conséquent de l'importance des pathologies liées à son dysfonctionnement, les composantes de cette voie telles que les protéines Smoothened, Patched (Patched 1 et Patched 2), les protéines Dispatched (Dispatched 1 et Dispatched 2) ou bien encore la protéine HIP représentent des cibles pour la mise au point de nouvelles molécules capables de moduler (activer ou inhiber) cette voie et donc de réguler positivement ou négativement le développement [prolifération, différenciation, migration, survie (apoptose)] et/ou l'activité de cellules différenciées et de cellules souches, *in vitro* et/ou *in vivo* chez l'embryon ou chez l'adulte.

De telles molécules sont utiles dans le traitement des tumeurs associées à une hyperactivation de la voie Hedgehog : les tumeurs du tissu nerveux (médulloblastomes, tumeurs primitives neuroectodermiques, glioblastomes, méningiomes et oligodendrogliomes), les tumeurs cutanées (carcinomes basocellulaires, trichoépithéliomes), les tumeurs des tissus musculaires et osseux (rhabdomyosarcomes, ostéosarcomes) et les tumeurs d'autres tissus (rein, vessie).

De telles molécules sont également utiles dans le traitement des pathologies de type neuro-dégénératif nécessitant un blocage de la voie Hedgehog (maladie de Parkinson, Chorée de Huntington, maladie d'Alzheimer, sclérose en plaques, maladie du motoneurone), et des maladies dans lesquelles le blocage de la voie de signalisation Hedgehog pourrait être bénéfique comme le diabète.

De telles molécules sont également utiles dans le traitement médical ou chirurgical (chirurgie plastique ou réparatrice, greffe de tissus ou d'organes) de nombreuses pathologies aiguës, subaiguës ou chroniques, génétiques ou acquises -impliquant un dysfonctionnement tissulaire lié à une dérégulation de la voie Hedgehog-, pour induire la formation, la régénération, la réparation et/ou l'augmentation de l'activité de tissus tels que de manière non-limitative : le tissu nerveux [système nerveux central (cerveau, cerveau) et périphérique (neurones sensoriels, moteurs, sympathiques], l'os, le cartilage, les testicules, le foie, la rate, l'intestin, le pancréas, les reins, les muscles lisses et squelettiques, le coeur, les poumons, la peau et le système pileux, les muqueuses, les cellules sanguines et les cellules du système immunitaire. A titre d'exemple non-limitatif de ces pathologies, on peut citer notamment les neuropathies et les maladies neuromusculaires associées, le diabète, l'alopécie, les brûlures, les ulcérations (peau et muqueuse) et les troubles de la spermatogénèse.

Différentes molécules, capables de moduler l'activité de la voie Hedgehog, ont été identifiées :
- les protéines Hedgehog et des polypeptides dérivés (fragments, variants..), notamment des agonistes et des antagonistes des protéines Hedgehog (Demande Internationale PCT WO O1/98344 au nom de BIOGEN) ; du fait de leur taille, ces protéines et les polypeptides dérivés ne peuvent pas passer la barrière hématoencéphalique et ne peuvent donc pas être administrés par voie systémique, notamment pour le traitement des tumeurs cérébrales liées à une hyperactivation de la voie de signalisation des protéines Hedgehog. En outre, de telles molécules sont difficiles à produire et à purifier et sont peu stables,
- des molécules organiques hétérocycliques (Demande Internationale PCT WO 01/74344 au nom de CURIS et Chen et al., PNAS, 2002, 99, 14071-14076),
- des molécules hétérocycliques azotées (Demandes Internationales PCT WO 01/19800, WO 01/26644 et WO 02/30421 au nom de CURIS et Kamenetsky et al., J. Biol., 2002, 1, 1-19), et
- des stéroïdes végétaux dérivés de *Veratrum* spp (Jervine, cyclopamine et cycloposine) et de *Solanum* spp. (solanidine), substitués en position 16, 17 ou 18 par une amine ou un dérivé d'amine, et du cholestérol : Brevet américain US 6,432,970 et Demandes Internationales PCT WO 99/52534 et WO 01/27135 au nom de JOHNS HOPKINS UNIVERSITY SCHOOL OF MEDICINE ; Brevet américain US 6,291,516 et Demandes Internationales PCT WO 00/41545 au nom de ONTOGENY et Demande Internationale PCT WO 02/30462 au nom de CURIS ; Talpale et al., Nature, 2000, 406, 1005-1009 ; Berman et al., Science, 2002, 297, 1559-1561. Toutefois, la cyclopamine est un agent tératogène à l'origine d'holoprosencéphalie et de cyclopie pour l'embryon chez les mammifères et l'absence de toxicité, pour les mammifères, des autres composés dérivés de stéroïdes végétaux n'a pas été démontrée.

Il ressort de ce qui précède qu'il n'existe actuellement pas de molécule efficace pour le traitement des états pathologique nécessitant une modulation de l'activité de la voie de signalisation des protéines Hedgehog dont l'absence de toxicité ait été vérifiée par des essais cliniques chez l'Homme. En conséquence, les Inventeurs se sont donné pour but de pourvoir à des composés qui répondent mieux aux besoins de la pratique, notamment en ce qu'ils sont dépourvus de toxicité chez l'Homme et donc potentiellement utilisables en thérapie humaine.

Le Brevet européen EP 0 057 115 et le Brevet américain US 4,386,085 au nom de ROUSSEL-UCLAF décrivent des composés 19-nor stéroïdes substitués en position 11β par un radical comprenant un atome d'azote, de phosphore ou de silicium, possédant des propriétés anti-glucocorticoïde, progestomimétique ou anti-progestomimétique, androgène ou anti-androgène. Du fait de ces propriétés, l'utilisation de ces composés stéroïdes est préconisée pour : lutter contre les troubles liés à une hypersécrétion de corticoïdes, notamment pour lutter contre le vieillissement en particulier contre l'hypertension, l'athérosclérose, le diabète, l'obésité ainsi que la dépression de l'immunité et l'insomnie, pour préparer des contraceptifs, pour lutter contre les dérèglements hormonaux, notamment les cancers hormonaux-dépendants, les aménorrhées, dysménorrhées et les insuffisances lutéales, ainsi que pour traiter les hypertrophies et le cancer de la prostate, l'hyperandrogénie, l'anémie, l'hirsutisme et l'acné.

Parmi ces composés, la mifépristone (17β-hydroxy 11β-(4-diméthylamino phényl) 17α-(prop-1-ynyl)estra-4,9-dien 3-one), également dénommée RU-486 ou RU-38486, est un 19-nor stéroïde substitué en position 11β, par un radical comprenant un atome d'azote qui possède une activité antagoniste du récepteur de la progestérone et également une forte affinité pour les récepteurs des glucocorticoïdes et une faible affinité pour les récepteurs des androgènes ; cette molécule a reçu une autorisation de mise sur le marché pour les interruptions volontaires de grossesse et des essais cliniques de phase II sont en cours pour le traitement, chez les femmes ménopausées, de cancers du sein métastasés.

En lien avec l'activité antagoniste du récepteur de la progestérone de la mifépristone et son affinité pour les récepteurs des glucocorticoïdes, son utilisation a été proposée pour le traitement de cancers tels que le méningiome, le leiomyome, le leiomyosarcosome, le cancer du sein, de l'endomètre et de l'ovaire, le cancer de la prostate, le cancer du foie, la leucémie, l'adénomyome utérin, le syndrome de Cushing et l'ostéosarcome (Koide et al. 1998 The J. of Reproductive Medecine, 43, 551-560, Weiss et al. 1993 Arch. of Family Medecine, vol. 2, n°1, 63-69 ; Haak et al. 1990 Lancet, vol. 336, n° 8707, 124-125 ; Bonelli R. M. 1992 Diskussionsforum Medezinische Ethik, n°6-7, 38-43 ; Olson et al. 1986 J. of Neurosurgery, vol. 65, n° 1, 99-107 ; Fryer et al. 2000 J. of Biol. Chem., vol. 275, n°23. 17771-17777 ; Kudawara et al. 2001 Europ. J. of Cancer, vol. 37, n°13, 1703-1708) ; de pathologies neurodégénératives telles que l'attaque cérébrale, la maladie de Parkinson, la chorée d'Huntington, la maladie du motoneurone, les traumatismes crâniens, la maladie d'Alzheimer et autres démences (Behl et al. 1997 Europ. J. of Neuroscience, vol. 9. n°5, 912-920 ; WO 00/24390 : WO 98/48784 ; Belanoff et al. 2002 J. of Molec. Neuroscience vol. 19, n°1/2, 201-206 ; WO 99/59596) ; et de maladies métaboliques telle que le diabète, en particulier, le diabète non insulino-dépendant, le syndrome X, l'hypertension, l'obésité, l'élimination inadéquate du glucose, l'hyperglycémie, l'hyperinsulinémie et de l'hypertriglycémie (US 4,386,085 ; WO 98/27986 ; Getty et al. 1997 Intl J. of Obesity, vol. 21, n°10, 865-873).

Les Inventeurs ont montré que la mifépristone et certains de ses dérivés qui diffèrent des stéroïdes dérivés de la cyclopamine précités en ce qu'ils ne possèdent pas de fonction amine primaire, secondaire ou tertiaire, ni de dérivés d'amine ou d'hétérocycle azoté, substitués en position 16, 17 et 18, sont de manière surprenante, actifs sur la voie de signalisation des protéines Hedgehog.

Du fait de son absence de toxicité chez l'Homme, la mifépristone qui est utilisée en thérapie humaine dans différents pays depuis de nombreuses années, représente une alternative intéressante à la cyclopamine et à ses dérivés, pour le traitement des états pathologiques nécessitant une modulation (activation ou inhibition) de l'activité de la voie de signalisation des protéines Hedgehog.

En conséquence, la présente invention a pour objet l'utilisation du (17β-hydroxy 11β-(4-diméthylamino phényl) 17α-(prop-1-ynyl)estra-4,9-dien 3-one (mifépristone) de formule : 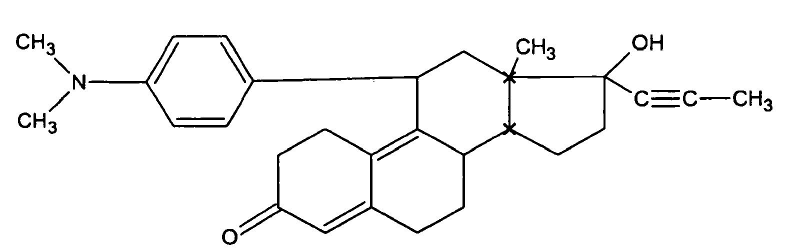 pour la préparation d'un médicament capable d'inhiber la voie de signalisation des protéines Hedgehog, destiné au traitement des tumeurs associées à une hyperactivation de cette voie de signalisation des protéines Hedgehog, sélectionnées dans le groupe constitué par : les médulloblastomes, les glioblastomes, les oligodendrogliomes, les carcinomes basocellulaires, les trichoépithéliomes, les rhabdomyosarcomes et les tumeurs du rein.

En particulier, le médicament selon l'invention est destiné à être administré par voie digestive, parentérale ou locale.

En outre, les inventeurs ont également décrit l'utilisation des composés de formule (I) 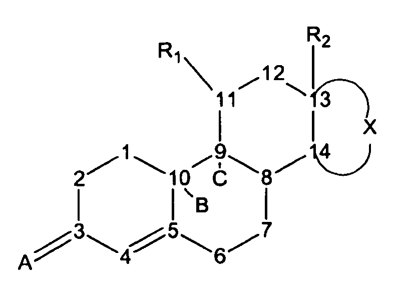 dans laquelle :
- R₁ représente un radical organique renfermant de 1 à 18 atomes de carbone, contenant au moins un atome d'azote, de phosphore ou de silicium, l'atome immédiatement adjacent au carbone 11 étant un atome de carbone,
- R₂ représente un radical hydrocarboné renfermant de 1 à 8 atomes de carbone,
- X de formule : 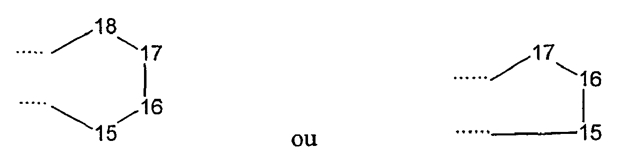 représente le reste d'un cycle pentagonal ou hexagonal, saturé ou insaturé, éventuellement substitué par un ou plusieurs groupements choisis parmi les radicaux : alkyle, alkényle, alkynyle, aryle, aralkyle en C₁-C₁₂, hydroxyle -OH, carbonyle 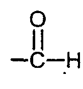 ou 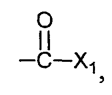 éther -O-X₂, ester 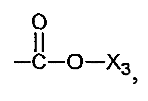 oxycarbonyle 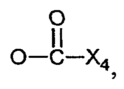 halogène, thiol, thioéther -S-X₅, sulfinyle 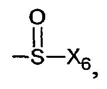 sulfonyle 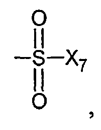 ainsi que les alkyle, akényle, alkynyle, aryle, aralkyle en C₁-C₁₂ substitués par une ou plusieurs fonctions hydroxyle -OH, carbonyle 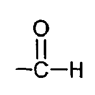 ou 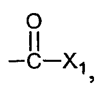 éther -O-X₂, ester 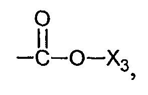 oxycarbonyle 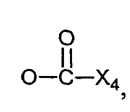 halogène, thiol, thioéther -S-X₅, sulfinyle 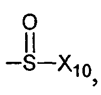 sulfonyle 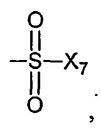 avec X₁, X₂, X₃, X₄, X₅, X₆ et X₇ représentant des groupements alkyle en C₁-C₈, alkényle, alkynyle en C₂-C₈ ou des groupements aryle, aralkyle en C₆-C₁₅,
- le groupement C= A en position 3, représente un groupement oxo, libre ou bloqué sous forme de cétal, un groupement alcool -CH-OH, éther -CH-O-Y₁, alkylcarboxylate 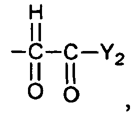 C=NOH, C=NO-Y₃ ou un groupement CH₂, Y₁, Y₂ et Y₃ représentant un radical alkyle renfermant de 1 à 8 atomes de carbone ou un groupement aralkyle renfermant de 7 à 15 atomes de carbone, et
- B et C forment ensemble une double liaison ou un pont époxyde, et des sels dérivés, pour la préparation d'un médicament capable de moduler (activer ou inhiber) la voie de signalisation des protéines Hedgehog, destiné au traitement des pathologies impliquant un dysfonctionnement tissulaire lié à une dérégulation de cette voie.

Parmi les sels dérivés on peut citer les sels d'addition avec les acides des composés de formule I, comme par exemple les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques, arylsulfoniques, tels que les acides méthane ou éthane sulfoniques, arylsulfoniques, tels que les acides benzène ou paratoluène sulfoniques et arylcarboxyliques.

Selon un mode de réalisation avantageux, R₂ représente un radical alkyle saturé, linéaire ou ramifié, renfermant de 1 à 4 atomes de carbone, de préférence un radical méthyle.

Selon un autre mode de réalisation avantageux, X représente un cycle pentagonal éventuellement substitué.

Selon une disposition avantageuse de ce mode de réalisation, ledit cycle pentagonal est substitué par au moins un groupement alkényle ou alkynyle, de préférence par un groupement alkynyle, de préférence en position 17.

Avantageusement, ledit cycle pentagonal est en outre substitué par au moins un groupement hydroxyle.

De manière préférée, X représente le reste d'un cycle pentagonal de formule :

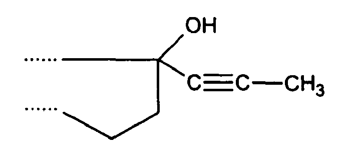

Selon encore un mode de réalisation avantageux, R₁ représente un radical hydrocarboné renfermant de 1 à 18 atomes de carbone et contenant au moins un atome d'azote, sélectionné parmi :
- les R₁ qui représentent un radical alkyle primaire, secondaire ou tertiaire, renfermant de 1 à 8 atomes de carbone, notamment un radical méthyle, éthyle, n-propyle, isopropyle, butyle, isobutyle, terbutyle, pentyle, hexyle, cyclopropyle, cyclobutyle, cyclopentyle ou cyclohexyle comportant au moins un atome d'azote ou substitué par un hétérocycle renfermant au moins un atome d'azote et éventuellement substitué par un radical alkyle renfermant de 1 à 8 atomes de carbone comme un radical méthyle, éthyle ou n-propyle, notamment les radicaux 3,4-ou 2-pyridyle, les radicaux thiazolyle ou pipéridinyle, et
- les R₁ qui représentent un radical aryle ou aralkyle portant une fonction amine, notamment un radical phényle ou benzyle portant une fonction amine : 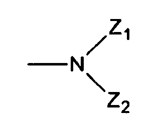 dans laquelle Z₁ et Z₂, identiques ou différents représentent un radical alkyle linéaire, ramifié ou cyclique renfermant de 1 à 8 atomes de carbone, Z₁ et Z₂ pouvant être éventuellement réunis pour former un hétérocycle avec l'atome d'azote. De manière préférée, Z₁ et Z₂ représentent un radical alkyle en C₁-C₄, et préférentiellement un radical méthyle.

Selon encore un autre mode de réalisation avantageux, le groupement C=A en position 3 représente un groupement oxo.

Selon encore un autre mode de réalisation avantageux, B et C forment ensemble une double liaison.

Selon une disposition avantageuse des modes de réalisation précédents, ledit composé de formule (I) est le (17β-hydroxy 11β-(4-diméthylamino phényl) 17α-(prop-1-ynyl)estra-4,9-dien 3-one de formule :

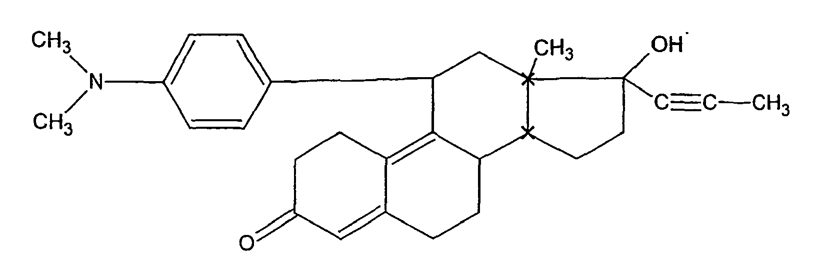

Les composés de formule I et leurs sels d'addition avec des acides pharmaceutiquement acceptables sont produits comme décrit dans les Brevets européen 0 057 115 et américain 4,386,085.

Les composés de formule I et leurs sels d'addition avec des acides pharmaceutiquement acceptables qui sont capables de moduler l'activité de la voie de signalisation des protéines Hedgehog sont utilisés dans le traitement des tumeurs associées à une hyperactivation de la voie Hedgehog, notamment les tumeurs du tissu nerveux (médulloblastomes, tumeurs primitives neuroectodermiques, glioblastomes, méningiomes et oligodendrogliomes), les tumeurs cutanées (carcinomes basocellulaires, trichoépithéliomes), les tumeurs des tissus musculaires et osseux (rhabdomyosarcomes, ostéosarcomes) et les tumeurs d'autres tissus (rein, vessie).

De tels composés sont également utilisés dans le traitement des pathologies nécessitant un blocage de la voie Hedgehog, notamment des pathologies de type neuro-dégénératif comme la maladie de Parkinson, la Chorée de Huntington, la maladie d'Alzheimer, la sclérose en plaques et la maladie du motoneurone ou bien d'autres pathologies dans lesquelles le blocage de la voie de signalisation Hedgehog pourrait être bénéfique, comme le diabète.

De tels composés sont encore utilisés dans le traitement médical ou chirurgical (chirurgie plastique ou réparatrice, greffe de tissus ou d'organes) de nombreuses pathologies aiguës, subaiguës ou chroniques, génétiques ou acquises- impliquant un dysfonctionnement tissulaire lié à une dérégulation de la voie Hedgehog-, pour induire, à partir de cellules souches ou de cellules différenciées, la formation, la régénération, la réparation et/ou l'augmentation de l'activité de tissus tels que de manière non-limitative : le tissu nerveux [système nerveux central (cerveau, cerveau) et périphérique (neurones sensoriels, moteurs, sympathiques], l'os, le cartilage, les testicules, le foie, la rate, l'intestin, le pancréas, les reins, les muscles lisses et squelettiques, le coeur, les poumons, la peau et le système pileux, les muqueuses, les cellules sanguines et du système immunitaire. A titre d'exemple non-limitatif de ces pathologies, on peut citer notamment les neuropathies et les maladies neuromusculaires associées, le diabète, l'alopécie, les brûlures, les ulcérations (peau et muqueuse) et les troubles de la spermatogénèse.

La posologie utile varie en fonction de l'affection à traiter, de la voie et du rythme d'administration, ainsi que de la nature et du poids de l'espèce à traiter (humaine ou animale) ; elle peut varier par exemple de 100 mg à 1 g par jour chez l'adulte par voie orale.

Les composés de formule (I) et leurs sels sont utilisés par voie digestive (orale, sublinguale), parentérale ou locale. Ils peuvent se présenter sous la forme de comprimés, simples ou dragéifiés, de gélules, de granules, de sirop, de suppositoires, de préparations injectables, de pommades, de crèmes, de gels, d'aérosol, lesquels sont préparés selon les méthodes usuelles.

Dans ces formes galéniques, les composés de formule (I) sont incorporés à des excipients habituellement employés dans des compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'utilisation des composés de formule (I) tels que définis ci-dessus et notamment de la mifépristone est plus avantageuse que celle des modulateurs de la voie de signalisation des protéines Hedgehog existants en ce que ces composés sont dépourvus de toxicité chez l'Homme et donc effectivement utilisables en thérapie humaine, contrairement à la cyclopamine et à ses dérivés, qui sont tératogènes.

Outre les dispositions qui précèdent, l'invention comprend encore d'autres dispositions qui ressortiront de la description qui va suivre, qui se réfère à des exemples de mise en oeuvre de la mifépristone et de ses dérivés selon la présente l'invention, ainsi qu'aux dessins annexés dans lesquels :

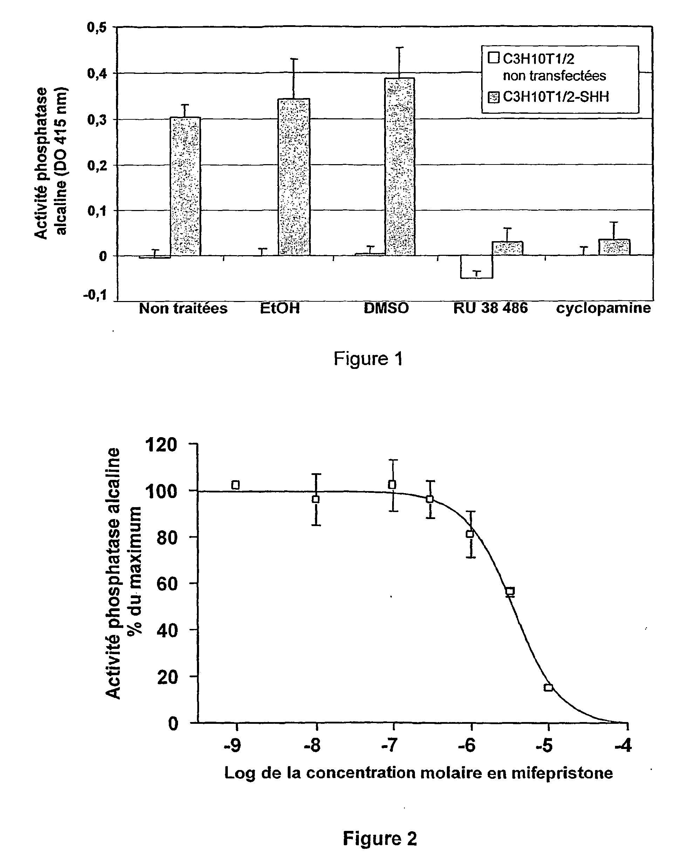

- la figure 1 illustre l'inhibition, par la mifépristone, de la différenciation de cellules mésenchymateuses (lignée C3H10T1/2) induite par la protéine Sonic Hedgehog (Shh). Des cellules C3H10T1/2 non-transfectées (colonnes blanches), et transfectées par le plasmide d'expression de la protéine Shh dénommé pRK5 (C3H10T1/2-SHH, colonnes grisées) sont traitées pendant 5 jours par la mifépristone (RU 38 486, 10 µM), la cyclopamine (10 µM) ou leurs véhicules respectifs, diméthylsulfoxyde (DMSO) ou éthanol (EtOH), ou non-traitées. La différenciation des cellules C3H10T1/2 qui s'accompagne d'une induction de l'activité phosphatase alcaline est ensuite évaluée par mesure de la densité optique à 415 nm, en présence du substrat de la phosphatase alcaline, le para-nitrophénylphosphate. Les valeurs indiquées correspondent à la moyenne ± erreur standard (n = 4), et
- la figure 2 illustre la courbe dose-effet de l'inhibition de la différenciation des cellules C3H10T1/2 par la mifépristone. Des cellules C3H10T1/2 transfectées par le plasmide d'expression de la protéine Shh dénommé pRK55, sont traitées pendant 5 jours par des quantités croissantes de mifépristone (RU 38 486, 1 nM à 10 µM) ou non-traitées. L'inhibition de la différenciation des cellules C3H10T1/2 est exprimée par le pourcentage d'activité phosphatase alcaline, par rapport aux cellules non-traitées (activité maximale = 100 %). Les valeurs indiquées correspondent à la moyenne ± erreur standard (n = 4) de deux expériences indépendantes. La concentration inhibant 50 % de la différenciation cellulaire (CI₅₀) est de 3,7 µM ± 0,3 µM.

### EXEMPLE : EFFET DE LA MIFEPRISTONE SUR LA VOIE DE SIGNALISATION DES PROTEINES HEDGEHOG

L'effet de la mifépristone sur la voie de signalisation des protéines Hedgehog est déterminé par l'analyse de la différenciation de la lignée de cellules fibroblastiques pluripotentes C3H10T1/2, préalablement induite par la protéine ShhN, en présence ou en l'absence de mifépristone ou de cyclopamine, pour comparaison.

### 1) Matériels et méthodes

La lignée de cellules fibroblastiques pluripotentes C3H10T1/2 (ATCC) est cultivée dans les conditions recommandées par l'ATCC. Des cellules C3H10T1/2 ont été transfectées par le plasmide pRK5 codant pour la protéine Shh de souris (Taipale et al., Nature, 2001, 411, 349-354), à l'aide de phosphate de calcium, selon les protocoles classiques. Ensuite, les cellules ont été cultivées pendant 5 jours en présence de mifépristone (10 µM), de cyclopamine (10 µM) ou bien de volumes correspondants de leur véhicules respectifs, diméthylsufoxide (DMSO) et éthanol (EtOH); puis l'activité phosphatase alcaline induite par la différenciation des cellules C3H10T1/2 en cellules musculaires, adipocytaires, chondrocytaires ou ostéoblastiques a été évaluée par mesure de la densité optique à 415 nm, en présence du substrat de la phosphatase alcaline, le para-nitrophénylphosphate. Alternativement, la courbe dose-réponse de la mifépristone a été déterminée dans les mêmes conditions expérimentales, en présence de concentrations croissantes de mifépristone (1 nM à 10 µM).

### 2) Résultats

### a) Effet comparatif de la mifépristone et de la cyclopamine sur la différenciation des cellules C3H10T1/2

Les résultats présentés à la figure 1 montrent que l'augmentation de l'activité phosphatase alcaline d'un facteur 3 à 10 qui est induite par la surexpression de Shh, est complètement inhibée en présence de 10 µM de mifépristone ou de cyclopamine.

Ces résultats démontrent que la mifépristone, qui contrairement à la cyclopamine est dépourvue d'effet tératogène, est capable comme la cyclopamine de bloquer la différenciation de cellules mésenchymateuses, induite par la protéine Shh. La mifépristone représente donc un antagoniste de la voie de signalisation des protéines Hedgehog, dépourvu d'effet tératogène, utile pour le traitement des pathologies nécessitant un blocage de la voie Hedgehog comme le cancer, les maladies neurodégénératives et le diabète.

### b) Courbe dose-effet de l'inhibition de la différenciation des cellules C3H10T1/2 par la mifépristone.

Les résultats présentés à la figure 2 qui confirment ceux présentés à la figure 1 montrent qu'une concentration de 10 µM de mifépristone inhibe complètement l'activation de la phosphatase alcaline induite par la surexpression de la protéine Shh. La concentration inhibant 50 % de la différenciation cellulaire (CI₅₀) est de 3,7 µM ± 0,3 µM.

## Revendications

1. Utilisation du composé 17β-hydroxy 11β-(4-diméthylaminophényl)17α-(prop-1-ynyl)estra-4,9-dien3-one de formule : 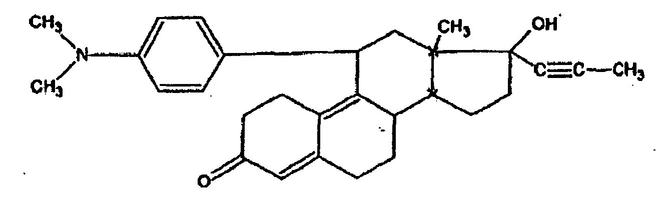 pour la préparation d'un médicament capable d'inhiber la voie de signalisation des protéines Hedgehog, destiné au traitement des tumeurs associées à une hyperactivation de cette voie de signalisation des protéines Hedgehog, sélectionnées dans le groupe constitué par : les médulloblastomes, les glioblastomes, les oligodendrogliomes, les carcinomes basocellulaires, les trichoépithéliomes, les rhabdomyosarcomes et les tumeurs du rein.

2. Utilisation selon la revendication 1, **caractérisée en ce que** ledit médicament est destiné à être administré par voie digestive, parentérale ou locale.

## Claims

1. Use of the compound 17β-hydroxy-11β-(4-dimethylaminophenyl)-17α-(prop-1-ynyl)estra-4,9-dien-3-one of the formula: 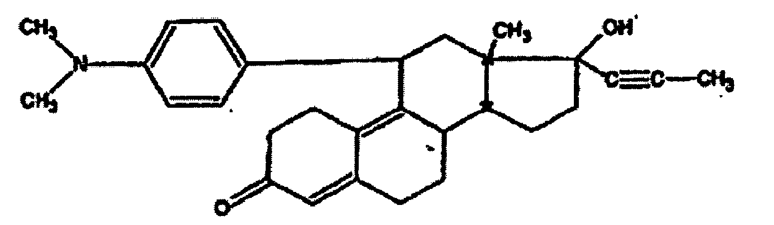 for the preparation of a medicament capable of inhibiting the signalling pathway of hedgehog proteins for treatment of tumours associated with hyperactivation of this hedgehog protein signalling pathway, selected from the group consisting of: medulloblastomas, glioblastomas, oligodendrogliomas, basocell carcinomas, trichoepitheliomas, rhabdomyosarcomas and kidney tumours.

2. Use according to claim 1, **characterized in that** the said medicament is intended for administration by the digestive, parenteral or local route.

## Patentansprüche

1. Verwendung der Verbindung 17β-Hydroxy-11β-(4-dimethylaminophenyl)-17α-(prop-1-inyl)estra-4,9-dien-3-on der Formel: 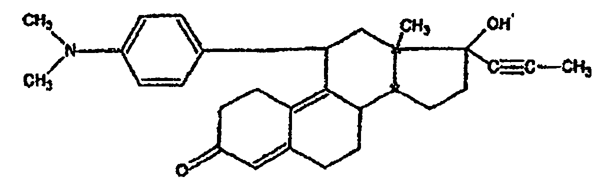 für die Herstellung eines Medikaments, das fähig ist, den Signalweg der Hedgehog-Proteine zu inhibieren, das zur Behandlung von Tumoren bestimmt ist, die mit einer Hyperaktivierung dieses Signalwegs der Hedgehog-Proteine assoziiert sind, die ausgewählt sind aus der Gruppe, bestehend aus: Medulloblastomen, Glioblastomen, Oligodendrogliomen, Basal-Zellkarzinomen, Trichoepitheliomen, Rhabdomyosarkomen und Tumoren der Niere.

2. Verwendung nach Anspruch 1, **dadurch gekennzeichnet, dass** das Medikament dazu bestimmt ist, auf digestivem, parenteralem oder lokalem Weg verabreicht zu werden.
